# EUROPÄISCHE PATENTSCHRIFT

(11) **EP 3 581 954 B1**
(45) Veröffentlichungstag und Bekanntmachung des Hinweises auf die Patenterteilung: **08.03.2023**
(21) Anmeldenummer: 18177237.7
(22) Anmeldetag: 12.06.2018
(51) Int. Cl.: G01R 33/36, G01R 33/3415, G01R 33/34

(54) **SENSOR UND MAGNETRESONANZTOMOGRAPH MIT DRAHTLOSER NAHFELDÜBERTRAGUNG VON ENERGIE UND DATEN**
SENSOR AND MAGNETIC RESONANCE TOMOGRAPHIC DEVICE WITH WIRELESS NEAR-FIELD TRANSMISSION OF POWER AND DATA
CAPTEUR ET TOMOGRAPHE À RÉSONANCE MAGNÉTIQUE À TRANSMISSION PAR CHAMP PROCHE SANS FIL D'ÉNERGIE ET DE DONNÉES

(43) Veröffentlichungstag der Anmeldung: 18.12.2019
(73) Patentinhaber: Siemens Healthcare GmbH, 91052 Erlangen (DE)
(72) Erfinder: Popescu, Stefan, 91056 Erlangen (DE)

(56) Entgegenhaltungen:
- DE-A1- 3 446 717
- DE-A1-102007 053 483
- DE-A1-102010 019 058
- DE-A1-102011 076 918
- DE-A1-102013 205 464

## Beschreibung

Die Erfindung betrifft einen Sensor, insbesondere eine Lokalspule, und einen Magnetresonanztomographen mit einer drahtlosen Übertragung von Energie und/oder Daten dazwischen.

Magnetresonanztomographen sind bildgebende Vorrichtungen, die zur Abbildung eines Untersuchungsobjektes Kernspins des Untersuchungsobjektes mit einem starken äußeren Magnetfeld ausrichten und durch ein magnetisches Wechselfeld zur Präzession um diese Ausrichtung anregen. Die Präzession bzw. Rückkehr der Spins aus diesem angeregten in einen Zustand mit geringerer Energie wiederum erzeugt als Antwort ein magnetisches Wechselfeld, auch als Magnetresonanzsignal bezeichnet, das über Antennen empfangen wird.

Mit Hilfe von magnetischen Gradientenfeldern wird den Signalen eine Ortskodierung aufgeprägt, die nachfolgend eine Zuordnung von dem empfangenen Signal zu einem Volumenelement ermöglicht. Das empfangene Signal wird dann ausgewertet und eine dreidimensionale bildgebende Darstellung des Untersuchungsobjektes bereitgestellt. Die erzeugte Darstellung gibt eine räumliche Dichteverteilung der Spins an.

Da die Magnetresonanzsignale sehr schwach sind, ist es üblich, Empfangsantennen, sogenannte Lokalspulen, möglichst nahe am zu untersuchenden Bereich anzuordnen. Zur Übertragung der empfangenen Signale und zur Stromversorgung von Vorverstärkern und Signalverarbeitung in den Lokalspulen werden dabei meist Kabelverbindungen zu dem Magnetresonanztomographen verwendet, die aber fehleranfällig, kompliziert zu handhaben und nicht zuletzt durch von den Anregungspulsen verursachten Mantelwellen auf den Leitern gefährlich für den Patienten sind, zumindest wenn entsprechende Sperrfilter ausfallen sollten.

Deshalb gibt es schon seit längerem Ansätze, die Lokalspulen mit drahtloser Übertragungstechnik zu versehen. Dabei ist aber zum einen die drahtlose Übertragungstechnik störanfällig, z.B. durch Interferenzen, eine Stromversorgung der Lokalspulen durch Batterien kann bei ständigem Einsatz zu Unterbrechungen für das Laden führen, und nicht zuletzt können die verwendeten Radiowellen die Magnetresonanzaufnahme stören.

In Verbindung mit dem Magnetresonanztomographen finden auch andere Sensoren Verwendung, wie beispielsweise EEG (Elektro-Enzephalogram), EKG (Elektro-Kardiogram) oder Atemsensor, die physiologische Daten während der Bildgebung erfassen und teilweise auch zur Steuerung der Bildgebung genutzt werden und die eine Energieversorgung benötigen.

Wird die Energieversorgung drahtlos ausgeführt, beispielsweise über die Körperspule, können die nötigen Leistungen den Patienten erwärmen und so die für die Aufnahme verfügbaren Leistungsbudgets verringern.

Aus dem Dokument DE 10 2013 205 464 A1 ist ein Lokalspulensystem (LS) zum Erfassen von MR-Signalen in einer MRT-Einrichtung bekannt. Das Lokalspulensystem weist eine Energieempfangsantenne zum induktiven Empfangen von Energie für das Lokalspulensystem aus einem sich zeitlich mit einer Energieübertragungsfrequenz ändernden Magnetfeld auf, wobei die Energieempfangsantenne eine schleifenartig von einem ersten Schleifenanschluss zu einem zweiten Schleifenanschluss verlaufende Leiterschleife umfasst.

Das Dokument DE 10 2010 019 058 A1 beschreibt eine Oberflächenspule ohne Versorgungs- und Datenleitungen. Durch eine resonante elektrische Streufeldkopplung zwischen den Dachkapazitäten von Sendemodulen und den Dachkapazitäten von Empfangsmodulen wird eine direkte Datenübertragung ohne Frequenzumsetzung oder Digitalisierung durchgeführt. Die Betriebsenergieversorgung der mobilen Sendemodule erfolgt drahtlos entweder direkt aus dem B1- Puls oder aus einem gesonderten hochfrequenten Speisefeld.

Das Dokument DE 34 46 717 A1 offenbart schließlich einen Kernspintomographen mit einer Hochfrequenzspulenanordnung zur Erzeugung eines hochfrequenten Magnetfeldes bzw. zum Empfang von Kernresonanzsignalen. Um Bewegungsartefakte zu vermeiden, wird die Impedanz der Hochfrequenzspulenanordnung auch während der eigentlichen Untersuchung gemessen, wobei aus Impedanzänderungen auf Bewegungen des Patienten geschlossen werden kann.

Es könnte daher eine Aufgabe der vorliegenden Erfindung sein, einen Sensor für einen sichereren und zuverlässigeren Betrieb bereitzustellen.

Die erfindungsgemäße Aufgabe wird durch einen Sensor nach Anspruch 1 und ein erfindungsgemäßes System aus Sensor und Magnetresonanztomograph nach Anspruch 7 gelöst.

Der erfindungsgemäße Sensor weist eine Energieversorgungseinrichtung, eine Datenübertragungseinrichtung und eine erste resonante Antenne auf. Als Energieversorgungseinrichtung wird gemäß der Erfindung eine Vorrichtung angesehen, die ausgelegt ist, aktive Komponenten des Sensors, wie beispielsweise bei einer Lokalspule Vorverstärker, lokale Oszillatoren, eine Steuerung, A/D-Wandler und Sicherheitskomponenten, mit Energie zu versorgen. Die Energieversorgungseinheit erhält dazu die notwendige Energie durch eine drahtlose Übertragung zwischen Magnetresonanztomograph und Sensor, wie sie nachfolgend näher erläutert wird. Die Energieversorgungseinrichtung kann beispielsweise Gleichrichter, Glättungskapazitäten, Spannungsregler bzw. Spannungswandler und auch Energiezwischenspeicher wie hochkapazitive Kondensatoren oder aufladbare Batterien aufweisen, um auch Zeiträume ohne oder mit reduzierter drahtloser Energieübertragung sowie Lastspitzen des Energieverbrauchs in dem Sensor ausgleichen zu können.

Eine Datenübertragungseinrichtung im Sinne der Erfindung ist ausgelegt, Daten von dem Sensor zu dem Magnetresonanztomographen und/oder in umgekehrter Richtung mittels einer drahtlosen Übertragung unter Verwendung hochfrequenter Signale mit einer Frequenz von mehr als 1 MHz, 10 MHz, 100 MHz oder 1 GHz zu übertragen. Die Daten können dabei analoge Signale wie vorverstärkte Magnetresonanzsignale, ggf. nach einer Frequenzumsetzung sein, die mit Amplituden- oder Frequenzmodulation oder einer anderen analogen Modulationstechnik auf die hochfrequenten Signale moduliert werden. Die Daten können aber auch digital sein wie Magnetresonanzsignale nach einer A/D-Wandlung. Die Datenübertragungseinrichtung kann im digitalen Fall dabei einen Datenpufferspeicher aufweisen und Codierungsverfahren verwenden, die ein Erkennen von Übertragungsunterbrechungen und nachfolgendes Wiederholen der Daten erlauben.

Die erste resonante Antenne steht in Signalverbindung mit der Datenübertragungseinrichtung. Die Signalverbindung kann beispielsweise ein Wellenleiter wie ein Koaxialkabel, symmetrischer Leiter oder eine Strichleitung sein. Dadurch können hochfrequente Signale von der Datenübertragungseinrichtung zu der resonanten Antenne geleitet werden oder umgekehrt von der resonanten Antenne zu der Datenübertragungseinrichtung.

Zwischen der Impedanz der Signalverbindung und der Impedanz der resonanten Antenne besteht bei einer ersten Resonanzfrequenz im freien Raum ohne nennenswerte Wechselwirkung mit einer anderen Antenne eine wesentliche Fehlanpassung. Das bedeutet, dass der Betrag des komplexen Widerstandes der Signalverbindung und der Antenne am Anschlusspunkt der ersten resonanten Antenne bei der ersten Frequenz beispielsweise um einen Faktor 2, 5, 10 oder mehr voneinander abweichen kann. Das aus der Fehlanpassung resultierende Stehwellenverhältnis kann größer als 2 oder 3 sein.

Eine fehlangepasste Antenne strahlt nur einen geringen Teil der zugeführten Leistung in eine sich im Raum ausbreitende freie elektromagnetische Welle ab. Stattdessen erzeugt beispielsweise eine fehlangepasste magnetische Antenne wie eine Antennenspule als Sendeantenne ein lokales magnetisches Wechselfeld, das mit der dritten Potenz des Abstandes in den Raum hinaus abfällt. Die Energie wechselt dabei von der Signalverbindung in das magnetische Nahfeld und zurück.

Wird eine resonante erste resonante Antenne in dieses Nahfeld eingebracht, wechselwirkt diese mit der Sendeantenne und entzieht dem (hier magnetischen) Wechselfeld Energie, die an einen Empfänger zum Empfang von Daten oder der Energieversorgungseinrichtung zugeführt werden kann. Durch die Wechselwirkung der ersten resonanten Antenne mit der Sendeantenne ändert sich auch die Impedanz der ersten resonanten Antenne und umgekehrt der Sendeantenne, sodass sich die Fehlanpassung bei der Energieübertragung oder Datenübertragung im Nahfeld wesentlich reduziert oder ganz verschwindet.

Der Sensor kann dabei insbesondere eine Lokalspule, aber auch ein Sensor zur Aufnahme eines EKG, EEG, Atmung oder anderer physiologischer Daten bzw. Messwerte sein.

Auf vorteilhafte Weise sorgt so die Fehlanpassung zwischen Signalverbindung und resonanter Antenne dafür, dass nur Energie in eine resonante Antenne übertragen wird und nicht unnötig abgestrahlt wird, insbesondere weil dadurch auch die SAR-Belastung erhöht wird. Darüber hinaus kann wegen der geringeren Abstrahlung auch eine Funkzulassung vereinfacht werden.

Vorzugsweise weist der Sensor eine Vielzahl an ersten Antennen auf, die insbesondere flächig nebeneinander angeordnet sind. Auf diese Weise kann sichergestellt werden, dass auch bei einer lateralen Verschiebung des Sensors z.B. auf der Patientenliege immer eine erste resonante Antenne im Nahbereich der nachfolgend erläuterten dritten Antenne(n) des Systems angeordnet ist.

Das erfindungsgemäße System weist einen Magnetresonanztomographen und einen erfindungsgemäßen Sensor auf. Der Magnetresonanztomograph weist eine Energiesendeeinrichtung und eine dritte resonante Antenne auf. Als Energiesendeeinrichtung im Sinne der Erfindung wird eine Einrichtung angesehen, die in der Lage ist, ein hochfrequentes Signal mit einer Resonanzfrequenz der dritten resonanten Antenne und einer Leistung ausreichend zur Versorgung des Sensors zu erzeugen. Die Energiesendeeinrichtung kann beispielsweise einen Oszillator und eine Leistungsendstufe aufweisen. Es ist auch denkbar, dass die Energiesendeeinrichtung das hochfrequente Signal zur Übertragung von Steuersignalen moduliert und die Energieversorgungseinrichtung des Sensors ausgelegt ist, das modulierte hochfrequente Signal zu demodulieren, um die Steuersignale in dem Sensor auszuwerten und umzusetzen.

Weiterhin weist der Magnetresonanztomograph eine Datenempfangseinrichtung auf. Die Datenempfangseinrichtung ist ausgelegt, von der Datenübertragungseinrichtung gesendete Daten zu empfangen. Bei analogen Daten kann das Empfangen beispielsweise ein Verstärken und demodulieren umfassen. Bei einem Frequenzmultiplex kann es darüber hinaus auch ein Demultiplexen umfassen. Für digitale Daten ist darüber hinaus ein Dekodieren und ggf. eine Fehlerkorrektur denkbar.

Die dritte resonante Antenne steht in Signalverbindung mit der Datenempfangseinrichtung. Die dritte resonante Antenne weist eine dritte Resonanzfrequenz auf, die im Wesentlichen mit der ersten Resonanzfrequenz der ersten Antenne übereinstimmt. Als wesentliche Übereinstimmung wird eine Abweichung kleiner als 5%, 1%, 0,5% oder 0,1% der dritten Resonanzfrequenz von der ersten Resonanzfrequenz angesehen. Bei der dritten Resonanzfrequenz besteht im freien Raum ohne nennenswerte Wechselwirkung mit einer anderen Antenne eine wesentliche Fehlanpassung zwischen der Impedanz der Signalverbindung am Anschlusspunkt der Signalverbindung an der dritten resonanten Antenne und der Impedanz der dritten resonanten Antenne bei der dritten Resonanzfrequenz. Zur wesentlichen Fehlanpassung gilt das bereits zum Sensor angeführte.

Durch die Wechselwirkung der ersten resonanten Antenne mit der dritten resonanten Antenne ändert sich auch die Impedanz der ersten resonanten Antenne und umgekehrt der dritten resonanten Antenne, sodass sich die Fehlanpassung bei der Energieübertragung oder Datenübertragung im Nahfeld wesentlich reduziert oder ganz verschwindet.

Auf vorteilhafte Weise erlaubt das System, den Sensor aufgrund der Fehlanpassung in einem Nahfeld der dritten resonanten Antenne mittels der ersten Antenne mit Energie zu versorgen oder Daten zu übertragen, ohne dabei in größerem Umfang Energie in den Raum abzustrahlen. Darüber hinaus ist durch die Nähe der Signalpegel größer und die Störsicherheit sowie Signal-zu-Rauschabstand (SNR) größer.

In einer denkbaren Ausführungsform des erfindungsgemäßen Sensors weist der Sensor eine zweite resonante Antenne auf. Die zweite resonante Antenne steht in Signalverbindung mit der Energieversorgungseinrichtung Die erste Resonanzfrequenz der ersten Antenne unterscheidet sich dabei wesentlich von der zweiten Resonanzfrequenz der zweiten Antenne. Die Abweichung kann dabei mehr als 5%, 10% oder 20% betragen, es können beide Antennen in unterschiedlichen Frequenzbändern wie MHz und GHz arbeiten, sodass sich die Resonanzfrequenzen um Größenordnungen unterscheiden. Vorzugsweise wird dabei die niedrigere Frequenz für die Energieübertragung zu der Energieversorgungseinrichtung mit der zweiten Antenne genutzt, da die Bandbreite zur Datenübertragung bei höheren Frequenzen größer ist.

Dabei besteht für die erste resonante Antenne bei der ersten Resonanzfrequenz und die zweite resonante Antenne bei der zweiten Resonanzfrequenz eine wesentliche Fehlanpassung zwischen der Impedanz der Signalverbindung und der Impedanz der zweiten resonanten Antenne. Zu der wesentlichen Fehlanpassung gilt das bereits gesagte.

Auf vorteilhafte Weise erlaubt es die zweite resonante Antenne mit der Fehlanpassung, neben der Energieübertragung auch eine Datenübertragung unabhängig und ohne Energieabstrahlung in den Raum vorzunehmen.

In einer möglichen Ausführungsform des erfindungsgemäßen Sensors steht die erste resonante Antenne in Signalverbindung mit der Energieversorgungseinrichtung und mit der Datenübertragungseinrichtung. Die erste resonante Antenne weist eine erste Resonanzfrequenz und eine davon unterschiedliche zweite Resonanzfrequenz auf, es handelt sich somit bei der ersten Antenne um eine doppelresonante Antenne. Dies kann beispielsweise erreicht werden, in dem in einer Leiterschleife einer Antenne beispielsweise ein Resonanzelement für zwei unterschiedliche Frequenzen vorgesehen wird.

Dabei gilt auch für die doppelresonante erste resonante Antenne, dass für die erste resonante Antenne eine wesentliche Fehlanpassung zwischen der Impedanz der Signalverbindung und der Impedanz der resonanten ersten Antenne bei der ersten Resonanzfrequenz und der zweiten Resonanzfrequenz besteht.

Auf vorteilhafte Weise erlaubt die doppeltresonante Antenne die Verwendung einer einzelnen Antenne für die Nahfeldübertragung von Energie und Daten.

In einer denkbaren Ausführungsform des erfindungsgemäßen Sensors ist die Datenübertragungseinrichtung ausgelegt, eine der ersten Antenne entnommene Energie zu modulieren. Es ist beispielsweise denkbar, dass die Datenübertragungseinrichtung in Signalverbindung zu der Energieversorgungseinrichtung steht, sodass die Datenübertragungseinrichtung steuern kann, wieviel Energie der ersten resonanten Antenne entzogen wird. Auf einer Sendeseite in dem Magnetresonanztomographen kann so die unterschiedliche Leistungsentnahme aus dem Feld detektiert werden und das übertragene Datensignal demoduliert werden. Auf vorteilhafte Weise kann so eine Datenübertragung realisiert werden, ohne dass der Sensor einen eigenen Sender aufweisen muss und insbesondere ohne Emission zusätzlicher Radiosignale.

In einer möglichen Ausführungsform eines erfindungsgemäßen Sensors weist der Sensor eine erste Hülle auf. Dies kann beispielsweise ein starres Gehäuse oder auch eine flexible und möglicherweise auch komprimierbare Hülle sein, um den Sensor an den Körper anzupassen und auch unter dem Patienten anordnen zu können. Dabei ist die erste resonante Antenne und/oder zweite resonante Antenne benachbart zu der Hülle auf einer Seite des Sensors angeordnet, die bei einem anwendungsgemäßen Betrieb des Sensors von einem Patienten abgewandt ist. Mit anderen Worten, die erste und/oder zweite resonante Antenne ist vorzugsweise an der Innenseite der Hülle derart angeordnet, dass sie einen möglichst geringen Abstand zu einer dritten Antenne des Magnetresonanztomographen hat, die über das Nahfeld Energie überträgt und/oder Daten sendet bzw. empfängt.

Auf vorteilhafte Weise kann durch die Anordnung der Antenne an oder in der Hülle ein besonders geringer Abstand zu einer Sendeantenne des Magnetresonanztomographen erreicht werden, der eine besonders effektive Übertragung von Energie und/oder Daten erlaubt.

In einer denkbaren Ausführungsform des erfindungsgemäßen Sensors weist der Sensor eine erste Hülle und eine von der ersten Hülle des Sensors getrennte zweite Hülle auf. In der zweiten Hülle ist die erste und/oder zweite resonante Antenne angeordnet. Die zweite Hülle ist mit einer elektrischen Leitung mit der ersten Hülle verbunden, sodass die erste und/oder zweite resonante Antenne mit der Energieversorgungseinrichtung und/oder der Datenübertragungseinrichtung elektrisch verbunden ist. Die zweite Hülle kann dabei auch eine zusätzliche Funktion erfüllen, beispielsweise als Befestigungsband bzw. Befestigungsmittel für den Sensor am Patienten oder der Patientenliege dienen.

Durch die in der zweiten Hülle angeordnete erste bzw. zweite resonante Antenne kann der Sensor weitgehend unabhängig von der Lage einer dritten Antenne des Magnetresonanztomographen am Patienten angeordnet werden, ohne die Energie und/oder Datenübertragung zu gefährden.

In einer möglichen Ausführungsform des erfindungsgemäßen Systems ist die dritte resonante Antenne in einem Bereich des Magnetresonanztomographen angeordnet, der sich bei anwendungsgemäßem Gebrauch des Sensors in unmittelbarer Nachbarschaft zu der ersten resonanten Antenne befindet. Beispielsweise kann die dritte resonante Antenne in einer Patientenliege im Bereich des Rückens des Patienten angeordnet sein, sodass beispielsweise eine Spine-Spule eine korrespondierende erste und/oder zweite resonante Antenne an der Unterseite (im Inneren des Gehäuses bzw. Hülle) aufweist, sodass die erste resonante Antenne und/oder zweite resonante Antenne in Wechselwirkung mit einem Nahfeld der dritten resonanten Antenne steht. Denkbar ist beispielsweise auch eine dritte resonante Antenne in oder hinter einer Verkleidung der Patientendurchführung, auch unter einer Patientenliege.

Auf vorteilhafte Weise wird durch die korrespondierende gegenüberliegende Anordnung der Antennen in Sensor und Magnetresonanztomograph ein optimaler Energietransfer und Datenaustausch sichergestellt.

In einer denkbaren Ausführungsform des erfindungsgemäßen System sind die bei anwendungsgemäßem Gebrauch zwischen der dritten resonanten Antenne und der ersten resonanten Antenne liegenden

Oberflächen eines Gehäuses des Magnetresonanztomographen und einer Hülle des Sensors derart komplementär ausgeformt, dass ein Abstand zwischen der dritten resonanten Antenne und der ersten resonanten Antenne im Wesentlichen minimal ist. Beispielsweise können beide Gehäuseoberflächen eben ausgeführt sein, oder eine konkav und die andere konvex. Es ist aber auch denkbar, dass beispielsweise eine Patientenliege eine ebene oder konkave Oberfläche aufweist und die Hülle des Sensors flexibel ist, sodass der Sensor unter dem Patienten an die Patientenliege angepresst wird.

Auf vorteilhafte Weise stellt eine korrespondierende Form der Hülle des Sensors und Oberfläche des Magnetresonanztomographen einen minimalen Abstand und so einen optimalen Energie- und Datentransfer sicher.

In einer möglichen Ausführungsform weist das erfindungsgemäße System eine Vielzahl an ersten resonanten Antennen und/oder zweiten resonanten Antennen und dritten resonanten Antennen auf. Vorzugsweise sind die Vielzahl der ersten resonanten Antennen und/oder zweiten resonanten Antennen dabei flächig an der Hülle des Sensors und die dritten resonanten Antennen flächig an der Oberfläche des Magnetresonanztomographen angeordnet, sodass sich bei anwendungsgemäßer Anordnung des Sensors an dem Patienten eine Mehrzahl an ersten resonanten Antennen und/oder zweiten resonanten Antennen jeweils im Nahbereich einer Mehrzahl an dritten resonanten Antennen befindet.

Die flächige Anordnung der Antennen an den gegenüberliegenden Flächen der Hülle bzw. Oberfläche stellt auf vorteilhafte Weise sicher, dass sich immer einige Paare an ersten/zweiten Antennen und dritten Antennen gegenüber stehen und so ein kontinuierlicher Transport an Energie und/oder Daten sichergestellt ist.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Es zeigen:
- Fig. 1: eine beispielhafte schematische Darstellung eines erfindungsgemäßen Magnetresonanztomographen mit einer erfindungsgemäßen Lokalspule;
- Fig. 2: eine beispielhafte schematische Darstellung einer Antenneneinheit und Hochfrequenzeinheit des erfindungsgemäßen Magnetresonanztomographen und einer erfindungsgemäßen Lokalspule;
- Fig. 3: Beispiele für Lokalspulen, die an Schulter, Knie oder Abdomen angeordnet werden können;
- Fig. 4: eine beispielhafte Anordnung für eine oder mehrere Lokalspulen und eine Antenneneinheit;
- Fig. 5: eine beispielhafte Ausführungsform einer doppeltresonanten Antenne;
- Fig. 6: eine beispielhafte Ausführungsform einer erfindungsgemäßen Lokalspule, bei der die Antennenspule, die erste resonante Antenne und die zweite resonante Antenne räumlich getrennt sind.

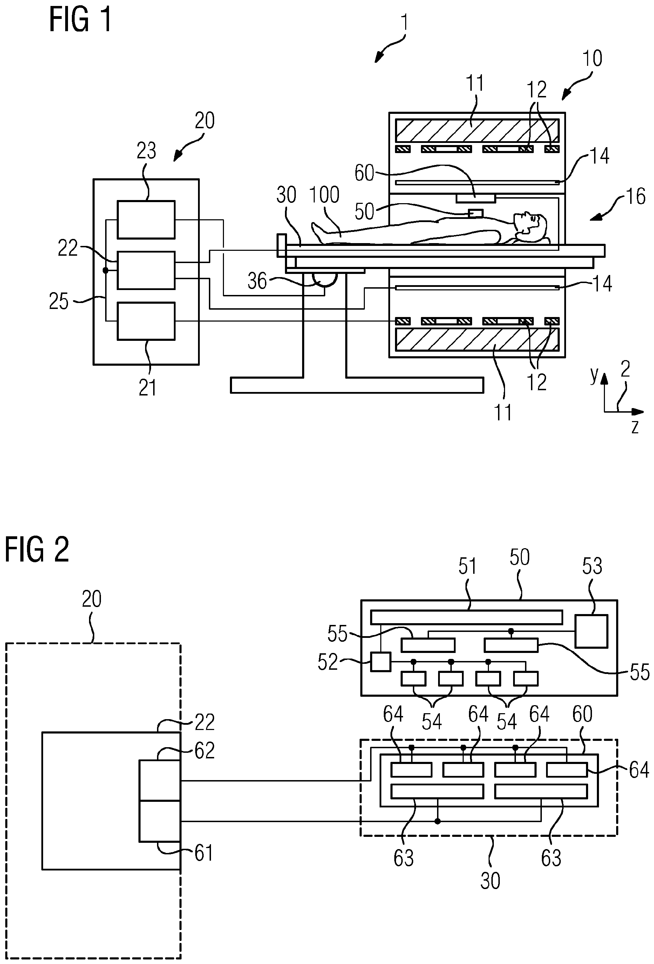

Fig. 1 zeigt eine schematische Darstellung einer Ausführungsform eines erfindungsgemäßen Magnetresonanztomographen 1 mit einer erfindungsgemäßen Lokalspule 50.

Die Magneteinheit 10 weist einen Feldmagneten 11 auf, der ein statisches Magnetfeld B0 zur Ausrichtung von Kernspins von Proben bzw. Patienten 100 in einem Aufnahmebereich erzeugt. Der Aufnahmebereich ist in einer Patientendurchführung 16 angeordnet, der sich in einer Längsrichtung 2 durch die Magneteinheit 10 erstreckt. Ein Patient 100 ist mittels der Patientenliege 30 und der Verfahreinheit 36 der Patientenliege 30 in den Aufnahmebereich verfahrbar. Üblicherweise handelt es sich bei dem Feldmagneten 11 um einen supraleitenden Magneten, der magnetische Felder mit einer magnetischen Flussdichte von bis zu 3T, bei neuesten Geräten sogar darüber, bereitstellen kann. Für geringere Feldstärken können jedoch auch Permanentmagnete oder Elektromagnete mit normalleitenden Spulen Verwendung finden.

Weiterhin weist die Magneteinheit 10 Gradientenspulen 12 auf, die dazu ausgelegt sind, zur räumlichen Differenzierung der erfassten Abbildungsbereiche in dem Untersuchungsvolumen dem Magnetfeld B0 variable Magnetfelder in drei Raumrichtungen zu überlagern. Die Gradientenspulen 12 sind üblicherweise Spulen aus normalleitenden Drähten, die zueinander orthogonale Felder in dem Untersuchungsvolumen erzeugen können.

Die Magneteinheit 10 weist ebenfalls eine Körperspule 14 auf, die dazu ausgelegt ist, ein über eine Signalleitung 33 zugeführtes Hochfrequenzsignal in das Untersuchungsvolumen abzustrahlen und von dem Patient 100 emittierte Resonanzsignale zu empfangen und über eine Signalleitung abzugeben. Bevorzugter Weise wird aber die Körperspule 14 für das Aussenden des Hochfrequenzsignals und/oder das Empfangen durch Lokalspule 50 ersetzt, die in der Patientendurchführung 16 nahe am Patient 100 angeordnet sind. Es ist aber auch denkbar, dass die Lokalspule 50 zum Senden und Empfangen ausgelegt ist und deshalb eine Körperspule 14 entfallen kann.

Eine Steuereinheit 20 versorgt die Magneteinheit 10 mit den verschiedenen Signalen für die Gradientenspulen 12 und die Körperspule 14 und wertet die empfangenen Signale aus. Eine Magnetresonanztomographen-Steuerung 23 koordiniert dabei die Untereinheiten.

So weist die Steuereinheit 20 eine Gradientenansteuerung 21 auf, die dazu ausgelegt ist, die Gradientenspulen 12 über Zuleitungen mit variablen Strömen zu versorgen, welche zeitlich koordiniert die erwünschten Gradientenfelder in dem Untersuchungsvolumen bereitstellen.

Weiterhin weist die Steuereinheit 20 eine Hochfrequenzeinheit 22 auf, die ausgelegt ist, einen Hochfrequenz-Puls mit einem vorgegebenen zeitlichen Verlauf, Amplitude und spektraler Leistungsverteilung zur Anregung einer Magnetresonanz der Kernspins in dem Patienten 100 zu erzeugen. Dabei können Pulsleistungen im Bereich von Kilowatt erreicht werden. Die einzelnen Einheiten sind über einen Signalbus 25 untereinander verbunden.

Das von der Hochfrequenzeinheit 22 erzeugte Hochfrequenzsignal wird über eine Signalverbindung der Körperspule 14 zugeführt und in den Körper des Patienten 100 ausgesendet, um dort die Kernspins anzuregen. Denkbar ist aber auch ein Aussenden des Hochfrequenzsignals über eine oder mehrere Spulenwicklungen der Lokalspule 50.

Die Lokalspule 50 empfängt dann vorzugsweise ein Magnetresonanzsignal aus dem Körper des Patienten 100, denn aufgrund des geringen Abstandes ist das Signal-zu-Rauschverhältnis (SNR) der Lokalspulen 50 besser als bei einem Empfang durch die Körperspule 14. Das von der Lokalspule 50 empfangene MR-Signal wird in der Lokalspule 50 aufbereitet und an die Hochfrequenzeinheit 22 des Magnetresonanztomographen 1 zur Auswertung und Bilderfassung weitergeleitet.

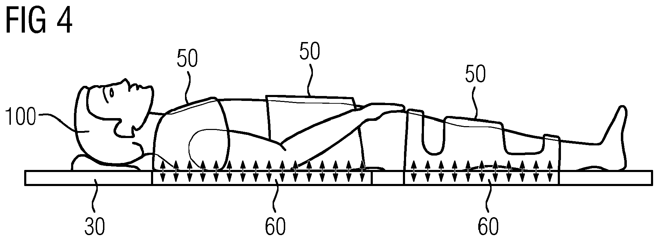

Die Signalverbindung zwischen Lokalspule 50 und Magnetresonanztomograph 1 ist dabei zumindest zwischen der Lokalspule 50 und der Antenneneinheit 60 drahtlos. Auf die Besonderheiten dieser drahtlosen Verbindung wird dabei in der Beschreibung zu den nachfolgenden Figuren näher eingegangen. Die Anordnung der Lokalspule 50 auf dem Patienten 100 und der Antenneneinheit 60 in der Patientendurchführung 16 ist nur beispielhaft und aus Gründen der Übersichtlichkeit der Zeichnung so gewählt. In einer nachfolgend in Fig. 4 dargestellten Ausführungsform ist beispielsweise die Lokalspule 50 unter dem Patienten 100 und die Antenneneinheit 60 in der Patientenliege 30 vorgesehen. Denkbar ist auch, dass die Antenneneinheit 60 in oder unter einer Verkleidung der Patientendurchführung 16 angeordnet ist. Insbesondere erfolgt die Energieversorgung der Lokalspule 50 zumindest zeitweise durch elektromagnetische Wechselwirkung mit der Antenneneinheit 60. Die Erzeugung der Signale von dem Magnetresonanztomographen 1 zu der Lokalspule 50 erfolgt durch die Hochfrequenzeinheit 22, die auch in umgekehrter Richtung Signale von der Lokalspule 50 über die Antenneneinheit 60 empfängt und auswertet.

In Verbindung mit einem Magnetresonanztomographen werden auch andere Sensoren, zum Beispiel zur Erfassung physiologischer Daten wie EEG, EKG oder Atmung genutzt. Beispielsweise kann mit einem Atemsensor oder einem EKG die Bildaufnahme mit der Bewegung der Organe synchronisiert werden, um Bildartefakte durch Bewegung zu minimieren. Auch diese Sensoren können wie die stellvertretend dargestellte Lokalspule 50 erfindungsgemäß drahtlos Energie und Daten von dem Magnetresonanztomographen 1 empfangen bzw. an diesen senden. In den nachfolgenden Ausführungsbeispielen wird jeweils eine Lokalspule 50 dargestellt, wobei die dort getroffenen Aussagen auch jeweils auf die anderen Arten von erfindungsgemäßen Sensoren anwendbar sind.

In Fig. 2 ist eine beispielhafte schematische Darstellung einer Antenneneinheit 60 und Hochfrequenzeinheit 22 des erfindungsgemäßen Magnetresonanztomographen 1 und einer erfindungsgemäßen Lokalspule 50 dargestellt. Die Größenverhältnisse sind dabei nicht maßstäblich.

Die Hochfrequenzeinheit 22 weist eine Energiesendeeinrichtung 61 auf, die beispielsweise einen Oszillator und einen Leistungsverstärker umfasst. Die Energiesendeeinrichtung 61 ist dabei ausgelegt, mit Hilfe der Antenneneinheit 60 ein magnetisches Wechselfeld zu erzeugen, über das die Lokalspule 50 drahtlos mit Energie versorgt werden kann.

In der dargestellten Ausführungsform weist die Hochfrequenzeinheit 22 darüber hinaus auch eine Datenempfangseinrichtung 62 auf, die ausgelegt ist, ein drahtloses Datensignal der Lokalspule 50 über die Antenneneinheit 60 zu empfangen und auszuwerten. Es ist dabei denkbar, dass die Datenempfangseinrichtung 62 auch ausgelegt ist, Daten zu der Lokalspule zur Steuerung zu übertragen oder dass die Hochfrequenzeinheit 22 darüber hinaus eine eigene Datenübertragungseinrichtung aufweist.

Die Hochfrequenzeinheit 22 ist über eine oder mehrere Signalverbindungen mit der Antenneneinheit 60 verbunden. Die Signalverbindung ist ausgelegt, das von der Energiesendeeinrichtung 61 erzeugte Hochfrequenzsignal zu einer oder mehreren dritten Antennen 63 zu leiten. Die Signalverbindung kann beispielsweise eine Koaxialleitung oder eine symmetrische Leitung (twisted pair, Stegleitung) mit definiertem Wellenwiderstand sein, sodass ein Signal oder auch mehrere Signale mit unterschiedlicher Frequenz über die Signalverbindung zu der Antenneneinheit 60 geleitet werden. Es ist so in einer Ausführungsform auch denkbar, ein Signal zur Energieversorgung auf einer Frequenz und ein anderes Signal zur Steuerung auf einer anderen Frequenz zu übertragen. Es wäre aber auch denkbar, dass die Energiesendeeinrichtung das Hochfrequenzsignal moduliert und so ein Steuersignal von der Steuereinheit 20 zu der Lokalspule 50 überträgt.

Die dritte resonante Antenne 63 weist eine wesentliche Fehlanpassung auf, sodass diese nahezu nur ein hochfrequentes Nahfeld, im Fall einer Sendespule als dritte resonante Antenne 63, ein vorwiegend magnetisches Wechselfeld im Nahfeld erzeugt, ohne dass Energie in größeren Umfang als elektromagnetische Welle in den Raum abgestrahlt wird. Beispielsweise kann die abgestrahlte Energie weniger als 20 %, 10%, 5% oder 1% der über die Signalverbindung zugeführten sein. Die übrige Energie wird reflektiert und beispielsweise in der Signalverbindung bzw. Zuleitung temporär gespeichert.

Die Fehlanpassung der dritten resonanten Antenne 63 kann zum einen erreicht werden, indem die Eingangsimpedanz der dritten resonanten Antenne 63 einen wesentlich anderen Wert aufweist als die Impedanz der Signalverbindung am Anschlusspunkt. Beispielsweise kann ein Koaxialkabel als Signalverbindung eine Impedanz von 50 Ohm aufweisen, während eine strahlende Antenne mit Fernfeld eine Freiluftimpedanz von 377 Ohm aufweist. Die Eingangsimpedanz der dritten resonanten Antenne 63 kann dabei beispielsweise über die umschlossene Fläche, die Anzahl der Spulenwindungen oder durch Abstimmkapazitäten variiert werden.

Es ist in einer Ausführungsform aber auch denkbar, dass die Antenneneinheit eine oder mehrere Anpassungselemente aufweist, die durch die Auslegung der Bauelemente gerade eine Fehlanpassung erzeugt, also im Sinne der Erfindung als "Fehl"-Anpassungselemente agieren. Dies kann beispielsweise bei geeigneter Wahl der Kapazitäten und Induktivitäten eines Pi-Gliedes erreicht werden. Auf vorteilhafte Weise kann so ein handelsübliches Hochfrequenz-Kabel als Signalverbindung verwendet werden, wobei durch die korrekte Anpassung der Impedanzen an beiden Endpunkten durch die Energiesendeeinrichtung und des Anpassungselements auf der Seite zum Kabel hin die Länge des Kabels unterschiedlich sein kann.

In einer anderen Ausführungsform wäre es aber auch denkbar, dass die Signalverbindung eine vorbestimmte Länge aufweist und dadurch selbst als Transformator bzw. Anpassungselement wirkt und eine Impedanz an der Energiesendeeinrichtung an einem Ende des Kabels zu einem unangepassten Impedanzwert am Anschlusspunkt der Antenne transformiert. Beispielsweise transformiert eine Leitung mit einer Länge, die einem Viertel der Wellenlänge auf der Leitung entspricht, eine sehr hohe Impedanz (offene Leitung) zu einer extrem niedrigen Impedanz (Kurzschluss) an ihrem anderen Ende. Auf diese Weise kann auf ein separates Anpassungselement in der Antenneneinheit 60 verzichtet werden.

In einer möglichen Ausführungsform weist die Antenneneinheit 60 eine vierte resonante Antenne 64 auf, die über eine Signalverbindung mit der Datenempfangseinrichtung 62 der Hochfrequenzeinheit 22 in Verbindung steht. Die Datenempfangseinrichtung 62 ist ausgelegt, über die vierte resonante Antenne 64 ein Magnetresonanzsignal von der Lokalspule zu empfangen. Das Magnetresonanzsignal kann dabei analog sein und dabei auf eine andere Frequenz umgesetzt sein, in Amplituden oder Frequenzmodulation auf eine Trägerwelle moduliert oder auch mit Magnetresonanzsignalen anderer Antennenspulen der Lokalspule über Frequenzmultiplex oder Zeitmultiplex gleichzeitig übertragen werden. Denkbar ist aber insbesondere auch eine Übertragung von digitalisierten Magnetresonanzsignalen, die dann durch Zwischenspeicherung und digitale Fehlerkorrektur auch bei kurzen Unterbrechungen der Verbindung vollständig übertragen werden können.

Vorzugsweise ist auch die vierte resonante Antenne 64 fehlabgestimmt. Auch wenn wegen der höheren Bandbreite vorzugsweise höhere Frequenzen als bei der Energieübertragung zum Einsatz kommen, gilt zur Fehlanpassung das bereits zur bei der Energieübertragung und der dritten resonanten Antenne 63 gesagte. Lediglich die Dimensionierung der Komponenten unterscheidet sich.

Für eine Übertragung von Steuerbefehlen in umgekehrter Richtung von dem Magnetresonanztomographen 1 zu der Lokalspule 50 über die Antenneneinheit 60 kann wie bereits erläutert das zur Energieübertragung genutzte magnetische Wechselfeld moduliert werden oder auch über die vierte resonante Antenne 64 ein bidirektionaler Verkehr von der Datenempfangseinrichtung erfolgen.

In Fig. 2 ist weiterhin eine erfindungsgemäße Lokalspule 50 dargestellt. Die Lokalspule 50 weist eine oder mehrere Antennenspulen 51 auf, die ausgelegt sind, ein Magnetresonanzsignal aus dem Körper des Patienten 100 zu empfangen. Die empfangenen Magnetresonanzsignale werden von einer Datenübertragungseinrichtung 52 für die Übertragung aufbereitet. Üblicherweise erfolgt zunächst eine Verstärkung durch einen rauscharmen Vorverstärker (LNB). Bei einer analogen Übertragung ist es dann denkbar, dass das Magnetresonanzsignal durch Mischen mit einem lokalen Oszillatorsignal auf eine andere Frequenz umgesetzt wird oder einem Träger mit Amplitudenmodulation oder Frequenzmodulation oder einem anderen Modulationsverfahren aufgeprägt wird. Denkbar ist auch ein Frequenzmultiplex mehrerer Signale. Bei einer digitalisierten Übertragung erfolgt zunächst eine Analog-Digital-Wandlung, bevor das digitalisierte Signal einem Träger aufmoduliert wird. Hier können die gleichen Modulationsverfahren wie bei der analogen Übertragung verwendet werden oder auch spezielle digitale wie beispielsweise Quadraturmodulation oder Spread-Spectrum-Verfahren. Vorzugsweise erfolgt vor der Übertragung eine Zwischenspeicherung in einem Puffer, um mittels Korrekturverfahren Störungen und Unterbrechungen auszugleichen und korrigieren zu können.

Erfindungsgemäß weist die Lokalspule eine oder mehrere erste resonante Antennen 54 auf, die in Signalverbindung mit der Datenübertragungseinrichtung stehen, sodass die Magnetresonanzdaten von der Datenübertragungseinrichtung wesentlich fehlabgestimmt sind. Zur Fehlabstimmung gilt das bereits zu der bzw. den dritten resonanten Antennen 63 gesagte. Auch hier wird durch die Fehlanpassung erreicht, dass das Signal der Datenübertragungseinrichtung vorwiegend in einem Nahfeld gespeichert wird statt als Raumwelle abgestrahlt zu werden.

Die Datenübertragungseinrichtung erfordert eine Zufuhr von Energie zum Betrieb, die durch eine Energieversorgungseinrichtung 53 erfolgt. Die Energieversorgungseinrichtung erhält die Energie über die zweite resonante Antenne 55, wenn diese sich im Nahfeld der dritten resonanten Antenne 63 befindet.

Dabei wird ein Wechselstrom in der zweiten resonanten Antenne induziert, der durch einen Gleichrichter, beispielsweise eine Diode, gleichgerichtet und durch eine Kapazität geglättet wird. Es ist darüber hinaus denkbar, dass ein Spannungswandler die Versorgungsspannung der Datenübertragungseinrichtung 52 konstant hält.

Um eine Energieversorgung sicherzustellen, auch wenn die Lokalspule bewegt wird und so kurzfristig die zweite resonante Antenne 55 das Nahfeld der dritten resonanten Antenne 63 verlässt, ist es denkbar, dass die Energieversorgungseinrichtung 53 einen Energiespeicher aufweist. Denkbar ist es, dass die Kapazität zum Glätten einen sehr großen Kapazitätswert aufweist, beispielsweise ein sogenannter Superkondensator ist. Möglich wäre auch eine aufladbare Batterie wie eine Lithium-Zelle, die über einen Laderegler geladen und entladen wird. Vorzugsweise ist dabei der Spannungsregler ein Step-Up-Converter, der auch bei niedriger Spannung der Kapazität in der Lage ist, eine höhere Ausgangsspannung konstant zu halten.

Zur Sicherstellung der Energieversorgung ist es weiterhin vorgesehen, dass die Lokalspule 50 und/oder die Antenneneinheit 60 jeweils eine Mehrzahl an zweiten resonanten Antennen 55 und dritten resonanten Antennen 63 aufweist, die entlang einer Achse bzw. über eine Fläche verteilt angeordnet sind, sodass bei einer Bewegung der Lokalspule, beispielsweise bei einer Bewegung des Patienten und anwendungsgemäßer Anordnung der Antenneneinheit immer jeweils eine zweite resonante Antenne 55 im Nahfeld einer dritten resonanten Antenne 63 angeordnet ist.

Das Gleiche gilt für die Übertragung der Magnetresonanzsignale mittels der ersten resonanten Antennen 54 und der vierten resonanten Antennen 64.

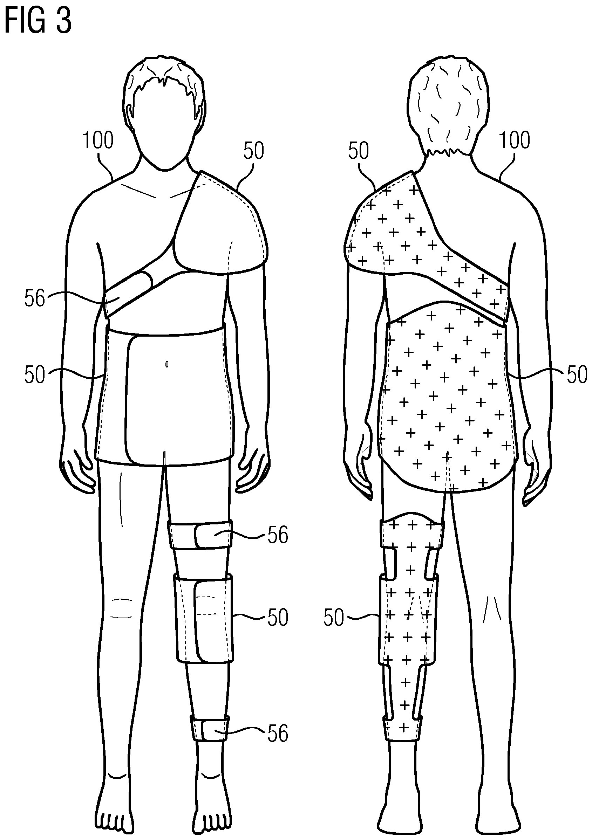

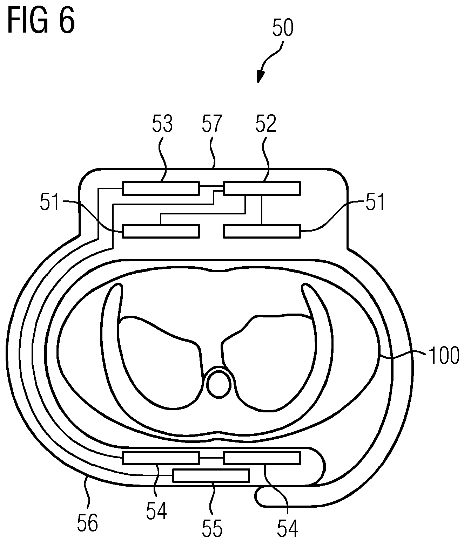

Fig. 3 gibt dazu Beispiele für Lokalantennen 50 an, die an Schulter, Knie oder Abdomen angeordnet werden können. Durch kleine Kreuze sind dabei Positionen von ersten resonanten Antennen 54 und zweiten resonanten Antennen 55 angedeutet. Die Antennenspulen 51 zum Empfang von Magnetresonanzsignalen aus dem Körper des Patienten 100 können dabei an beliebigen Stellen der Lokalspule 50 angeordnet sein, sodass sie Signale aus dem gesamten zu untersuchenden Volumen erfassen. Es muss lediglich eine Signalverbindung zwischen den Antennenspulen 51 zu der Datenübertragungseinrichtung 52 bestehen, sodass die Magnetresonanzsignale aufbereitet und zu dem Magnetresonanztomographen 1 übertragen werden können. Ein Beispiel einer räumlich getrennten Anordnung von Antennenspule 51 und ersten resonanten Antennen 54 und zweiten resonanten Antennen 55 ist in Fig. 6 dargestellt.

Fig. 4 stellt eine beispielhafte Anordnung für eine oder mehrere Lokalspulen 50 und eine Antenneneinheit 60 dar, wie sie bei einer erfindungsgemäßen Verwendung der Lokalspule 50 vorgesehen ist.

Die ersten resonanten Antennen 54 und zweiten resonanten Antennen 55 sind dabei derart in einer Hülle der Lokalspule 50 angeordnet, dass sie bei einer ordnungsgemäßen Verwendung der Lokalspule 50 möglichst nahe an einer korrespondierenden dritten resonanten Antenne 63 bzw. vierten resonanten Antenne 64 angeordnet sind. Bei einer Lokalspule 50, die beispielsweise wie eine Spine-Spule unter dem Patienten 100 zu liegen kommt, sind die zweiten resonanten Antennen 55 an der Unterseite der Hülle der Lokalspule 50 angeordnet, sodass sie möglichst nahe an den dritten resonanten Antennen 63 einer Antenneneinheit 60 zu liegen kommen, die beispielsweise in einer Patientenliege 30 angeordnet ist.

Dabei ist es auch denkbar, dass die Lokalspule 50 einen Teil der ersten Hülle 57 bzw. zweiten Hülle aufweist, der abgesetzt von der ersten Hülle mit den Antennenspulen 51 für das Magnetresonanzsignal ist und in elektrischer Verbindung mit diesem steht. Diese zweite Hülle könnte beispielsweise auch ein Befestigungselement wie eine Lasche 56 oder Schlaufe der Lokalspule 50 sein, mit der diese an dem Patienten befestigt wird. In dieser zweiten Hülle sind dann die ersten resonanten Antennen 54 und/oder die zweiten resonanten Antennen 55 angeordnet, sodass sie auch dann im Nahfeld der dritten resonanten Antennen 63 und/oder der vierten resonanten Antennen 64 sind, wenn die Lokalspule 50 mit den Antennenspulen 51 auf dem Körper des Patienten 100 angeordnet ist.

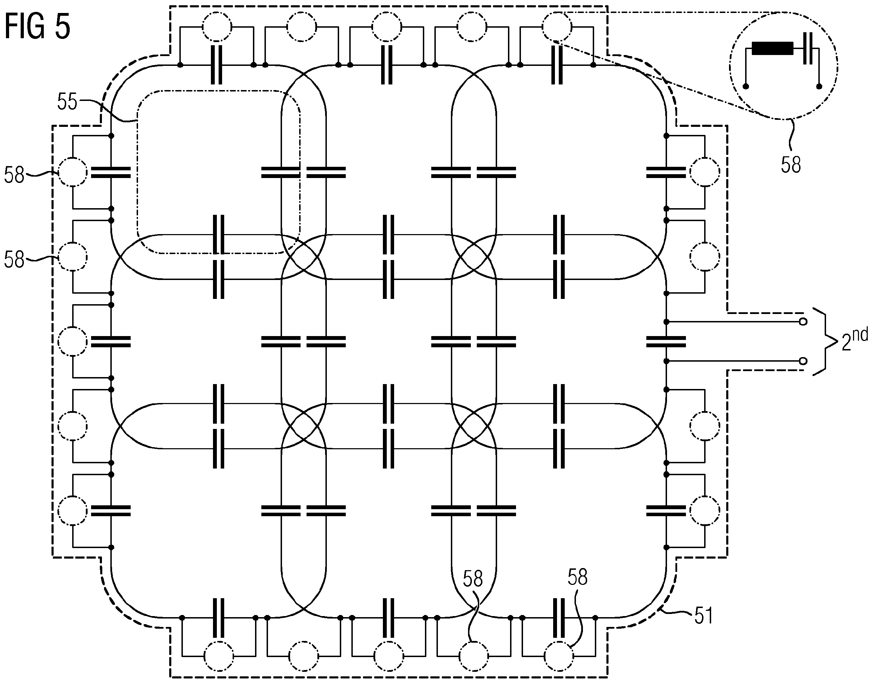

Fig. 5 zeigt eine beispielhafte Ausführungsform einer doppelt-resonanten Antenne. Die doppelt-resonante Antenne weist eine Vielzahl von kleineren Antennenschleifen auf, deren Verlauf exemplarisch für eine Schleife durch die Linie mit dem Punkt-Strich-Muster angedeutet ist, die beispielsweise als zweite resonante Antenne 55 zur Energieübertragung genutzt werden kann. Denkbar ist aber auch eine Nutzung als erste resonante Antenne 54 zur Datenübertragung. Die Länge der Antennenschleife wird elektrisch durch Verlängerungskondensatoren verlängert.

Gleichzeitig bilden die äußeren Leiterstücke der kleineren Antennenschleifen, wie durch die gestrichelte Linie angedeutet, eine Antennenspule 51 zur Aufnahme von Magnetresonanzsignalen aus dem Körper des Patienten 100. Dabei erfolgt die Verbindung der einzelnen Leiterstücke durch Resonanzelemente 58, hier als Serienresonanzkreis, die bei der Larmorfrequenz des Magnetresonanztomographen resonant sind. Entsprechende Resonanzelemente 58 überbrücken auch die Verlängerungskondensatoren.

Denkbar ist es auch, die Funktionen der ersten resonanten Antenne 54 zur Datenübertragung und der zweiten resonanten Antenne 55 zur Energieübertragung in einer doppelt-resonanten Antenne auf diese Weise zu vereinen oder alle drei Antennenarten der Lokalspule 50 in einer dreifach-resonanten Antenne nach dem gleichen Prinzip zu vereinen.

Fig. 6 stellt noch einmal ein Beispiel dar, bei dem die Antennenspule 51 und die erste resonante Antenne 54 und die zweite resonante Antenne 55 räumlich getrennt sind. Das Bild zeigt eine erfindungsgemäße Lokalspule für das Abdomen schematisch im Querschnitt. Die Lokalspule 50 ist auf der Bauchdecke des Patienten 100 angeordnet. In der ersten Hülle befinden sich die Antennenspulen 51 für den Empfang des Magnetresonanzsignals, die Datenübertragungseinrichtung 52 und die Energieversorgungseinrichtung 53. Als Befestigungselemente erstrecken sich seitlich Laschen 56 um den Patienten, die beispielsweise mit Klettverschlüssen geschlossen sind. In der Lasche 56 befinden sich auf der Unterseite des Patienten 100 erste resonante Antennen 54 und eine zweite resonante Antenne 55, die über elektrische Verbindungen mit der Datenübertragungseinrichtung 52 bzw. der Energieversorgungseinrichtung 53 verbunden sind. Die Lasche 56 bildet somit eine zweite Hülle, in der die ersten resonanten Antennen 54 und die zweiten resonanten Antennen 55 angeordnet sind. Auf diese Weise sind die erste resonante Antenne 54 und die zweite resonante Antenne 55 im Nahfeld einer dritten resonanten Antenne 63 bzw. vierten resonanten Antenne 64, wenn der Patient 100 wie in Fig. 2 und Fig. 4 dargestellt anwendungsgemäß auf der Patientenliege 30 liegt.

Dabei ist es auch denkbar, dass ein Sensor für ein EKG auf die gleiche Weise angeordnet wird. Anstelle der Antennenspule 51 treten dann jedoch Elektroden zur Erfassung der Muskelpotentiale des Herzens. Ebenso kann ein Atemsensor ausgebildet sein, der beispielsweise durch die Ausdehnung beim Atmen den Atemrhythmus erfasst.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den durch die Ansprüche definierten Schutzumfang der Erfindung zu verlassen.

## Patentansprüche

1. Sensor zur Verwendung in einem Magnetresonanztomographen, wobei der Sensor eine Energieversorgungseinrichtung (53), eine Datenübertragungseinrichtung (52) und eine erste resonante (54) Antenne aufweist, wobei die Energieversorgungseinrichtung (53) dazu eingerichtet ist, drahtlos mit Energie durch den Magnetresonanztomographen versorgt zu werden,
wobei die Datenübertragungseinrichtung (52) dazu eingerichtet ist, Daten drahtlos zwischen Sensor und dem Magnetresonanztomographen unter Verwendung hochfrequenter Signale mit einer Frequenz von mehr als 1 MHz zu übertragen,
wobei die erste resonante Antenne (54) in Signalverbindung mit der Datenübertragungseinrichtung (52) steht und eine wesentliche Fehlanpassung zwischen der Impedanz der Signalverbindung und der Impedanz der ersten resonanten Antenne (54) im freien Raum ohne nennenswerte Wechselwirkung mit einer anderen Antenne bei einer ersten Resonanzfrequenz der ersten resonanten Antenne (54) besteht, wodurch die erste resonante Antenne (54) dazu ausgelegt ist, nur einen geringen Teil der zugeführten Leistung in eine sich im Raum ausbreitende freie elektromagnetische Welle abzustrahlen.

2. Sensor nach Anspruch 1, wobei der Sensor eine zweite resonante Antenne (55) mit einer zweiten Resonanzfrequenz aufweist, wobei die zweite resonante Antenne (54) in Signalverbindung mit der Energieversorgungseinrichtung (53) steht, wobei die erste Resonanzfrequenz der ersten resonanten Antenne (54) sich wesentlich von der zweiten Resonanzfrequenz der zweiten resonanten Antenne (55) unterscheidet, wobei für die zweite resonante Antenne (55) bei der zweiten Resonanzfrequenz eine wesentliche Fehlanpassung zwischen der Impedanz der Signalverbindung und der Impedanz der zweiten resonanten Antenne (55) besteht.

3. Sensor nach Anspruch 1, wobei die erste resonante Antenne (54) in Signalverbindung mit der Energieversorgungseinrichtung (53) und mit der Datenübertragungseinrichtung (52) steht, wobei die erste resonante Antenne (54) eine erste Resonanzfrequenz und eine davon unterschiedliche zweite Resonanzfrequenz aufweist, wobei für die erste resonante Antenne (54) eine wesentliche Fehlanpassung zwischen der Impedanz der Signalverbindung und der Impedanz der erste resonanten Antenne (54) bei der ersten Resonanzfrequenz und der zweiten Resonanzfrequenz besteht.

4. Sensor nach Anspruch 1, wobei die Datenübertragungseinrichtung (52) ausgelegt ist, eine der ersten resonanten Antenne (54) entnommene Energie zu modulieren.

5. Sensor nach einem der vorhergehenden Ansprüche, wobei der Sensor eine erste Hülle (57) aufweist und die erste resonante Antenne (54) und/oder zweite resonante Antenne (55) benachbart zu der ersten Hülle (57) auf einer Seite des Sensors angeordnet ist, die bei einem anwendungsgemäßen Betrieb des Sensors von einem Patienten (100) abgewandt ist.

6. Sensor nach einem der Ansprüche 1 bis 5, wobei der Sensor eine von einer ersten Hülle (57) des Sensors getrennte zweite Hülle aufweist, die mit einer elektrischen Leitung mit der ersten Hülle (57) verbunden ist und in der die erste resonante Antenne (54) und/oder zweite resonante Antenne (55) angeordnet ist.

7. System aus einem Magnetresonanztomographen (1) und einem Sensor nach einem der vorhergehenden Ansprüche, wobei der Magnetresonanztomograph (1) eine Energiesendeeinrichtung (61), eine Datenempfangseinrichtung (62) und eine dritte resonante Antenne (63) mit einer dritten Resonanzfrequenz aufweist, wobei die dritte resonante Antenne (63) in Signalverbindung mit der Datenempfangseinrichtung (62) steht und die dritte Resonanzfrequenz der dritten resonanten Antenne (63) mit der ersten Resonanzfrequenz der ersten resonanten Antenne (55) im Wesentlichen übereinstimmt und ohne nennenswerte Wechselwirkung zwischen erster resonanter Antenne (54) und dritter resonanter Antenne (63) eine wesentliche Fehlanpassung zwischen der Impedanz der Signalverbindung und der Impedanz der dritten resonanten Antenne (63) bei der dritten Resonanzfrequenz besteht, wobei das System ausgelegt ist, durch die Wechselwirkung der ersten resonanten Antenne mit der dritten resonanten Antenne im Nahfeld die Impedanz der ersten resonanten Antenne und umgekehrt der dritten resonanten Antenne zu ändern, sodass sich die Fehlanpassung bei der Energieübertragung oder Datenübertragung im Nahfeld wesentlich reduziert oder ganz verschwindet und in einem Nahfeld der dritten resonanten Antenne mittels der ersten Antenne Daten zu übertragen, ohne dabei in größerem Umfang Energie in den Raum abzustrahlen.

8. System nach Anspruch 7, wobei die dritte resonante Antenne (63) in einem Bereich des Magnetresonanztomographen (1) angeordnet ist, der sich bei anwendungsgemäßem Gebrauch des Sensors in unmittelbarer Nachbarschaft zu der ersten resonanten Antenne (54) befindet.

9. System nach Anspruch 8, wobei bei anwendungsgemäßem Gebrauch zwischen der dritten resonanten Antenne (63) und der ersten resonanten Antenne (54) liegende Oberflächen eines Gehäuses des Magnetresonanztomographen (1) und einer Hülle des Sensors derart komplementär ausgeformt sind, dass ein Abstand zwischen der dritten resonanten Antenne (63) und der ersten resonanten Antenne (54) im Wesentlichen minimal ist.

10. System nach einem der Ansprüche 7 bis 9, wobei das System eine Vielzahl an ersten resonanten Antennen (54) und dritten resonanten Antennen (63) aufweist.

## Claims

1. Sensor for use in a magnetic resonance tomography unit,
wherein the sensor has an energy supply device (53), a data transmission device (52) and a first resonant (54) antenna, wherein the energy supply device (53) is configured to be wirelessly supplied with energy by the magnetic resonance tomography unit,
wherein the data transmission device (52) is configured to transmit data wirelessly between the sensor and the magnetic resonance tomography unit by means of radio-frequency signals with a frequency of more than 1 MHz,
wherein the first resonant antenna (54) has a signal connection to the data transmission device (52) and there exists a significant mismatch between the impedance of the signal connection and the impedance of the first resonant antenna (54) in free space without any appreciable interaction with another antenna at a first resonant frequency of the first resonant antenna (54), by which means the first resonant antenna (54) is configured to radiate only a small part of the power supplied to it into a free electromagnetic wave propagating in space.

2. Sensor according to claim 1, wherein the sensor has a second resonant antenna (55) with a second resonant frequency, wherein the second resonant antenna (54) has a signal connection to the energy supply device (53), wherein the first resonant frequency of the first resonant antenna (54) differs significantly from the second resonant frequency of the second resonant antenna (55), wherein for the second resonant antenna (55) there exists a significant mismatch between the impedance of the signal connection and the impedance of the second resonant antenna (55) at the second resonant frequency.

3. Sensor according to claim 1, wherein the first resonant antenna (54) has a signal connection to the energy supply device (53) and to the data transmission device (52), wherein the first resonant antenna (54) has a first resonant frequency and a second resonant frequency different therefrom, wherein for the first resonant antenna (54) there exists a significant mismatch between the impedance of the signal connection and the impedance of the first resonant antenna (54) at the first resonant frequency and the second resonant frequency.

4. Sensor according to claim 1, wherein the data transmission device (52) is designed to modulate energy obtained from the first resonant antenna (54).

5. Sensor according to one of the preceding claims, wherein the sensor has a first envelope (57) and the first resonant antenna (54) and/or second resonant antenna (55) is arranged adjacent to the first envelope (57) on a side of the sensor that, when the sensor is operating as per application, is facing away from a patient (100).

6. Sensor according to one of claims 1 to 5, wherein the sensor has a second envelope separated from a first envelope (57) of the sensor, which is linked by an electrical line to the first envelope (57) and in which the first resonant antenna (54) and/or second resonant antenna (55) is arranged.

7. System consisting of a magnetic resonance tomography unit (1) and a sensor according to one of the preceding claims, wherein the magnetic resonance tomography unit (1) has an energy transmission device (61), a data receiving device (62) and a third resonant antenna (63) with a third resonant frequency, wherein the third resonant antenna (63) has a signal connection to the data receiving device (62) and the third resonant frequency of the third resonant antenna (63) essentially matches the first resonant frequency of the first resonant antenna (55) and without appreciable interaction between first resonant antenna (54) and third resonant antenna (63) a significant mismatch between the impedance of the signal connection and the impedance of the third resonant antenna (63) exists at the third resonant frequency, wherein the system is configured to change the impedance of the first resonant antenna through the interaction of the first resonant antenna with the third resonant antenna in the near field and conversely that of the third resonant antenna, so that the mismatch during the energy transmission or data transmission in the near field significantly reduces or disappears entirely and to transmit data in a near field of the third resonant antenna by means of the first antenna, without radiating energy into the space to any great extent.

8. System according to claim 7, wherein the third resonant antenna (63) is arranged in an area of the magnetic resonance tomography unit (1) that, when the sensor is being used as per the application, is located in the immediate vicinity of the first resonant antenna (54).

9. System according to claim 8, wherein, when being used as per application, there are surfaces of the magnetic resonance tomography unit (1) and an envelope of the sensor formed complementarily lying between the third resonant antenna (63) and the first resonant antenna (54) in such a way that a distance between the third resonant antenna (63) and the first resonant antenna (54) is essentially minimal.

10. System according to one of claims 7 to 9, wherein the system has a plurality of first resonant antennas (54) and third resonant antennas (63).

## Revendications

1. Capteur à utiliser dans un tomodensitomètre à résonnance magnétique, dans lequel le capteur a un dispositif (53) d'alimentation en énergie, un dispositif (52) de transmission de données et une première antenne résonnante (54), dans lequel le dispositif (53) d'alimentation en énergie est conçu pour être alimenté sans fil en énergie par le tomodensitomètre à résonnance magnétique,
dans lequel le dispositif (52) de transmission de données est conçu pour transmettre sans fil des données entre le capteur et le tomodensitomètre à résonnance magnétique en utilisant des signaux de haute fréquence d'une fréquence de plus de 1 MHz, dans lequel, la première antenne (54) résonnante est en liaison de signal avec le dispositif (52) de transmission de données, et il y a une désadaptation sensible entre l'impédance de la liaison de signal et l'impédance de la première antenne (54) résonnante dans l'espace libre sans interaction notable avec une autre antenne à une première fréquence de résonnance de la première antenne (54) résonnante,
grâce à quoi la première antenne (54) résonnante est conçue pour rayonner seulement une partie petite de la puissance apportée dans une onde électromagnétique libre se propageant dans l'espace.

2. Capteur suivant la revendication 1, dans lequel le capteur a une deuxième antenne (55) résonnante ayant une deuxième fréquence de résonnance, la deuxième antenne (54) résonnante étant en liaison de signal avec le dispositif (53) d'alimentation en énergie, la première fréquence de résonnance de la première antenne (54) résonnante étant sensiblement différente de la deuxième fréquence de résonnance de la deuxième antenne (55) résonnante, dans lequel, pour la deuxième antenne (55) résonnante à la deuxième fréquence de résonnance, il y a une désadaptation sensible entre l'impédance de la liaison de signal et l'impédance de la deuxième antenne (55) résonnante.

3. Capteur suivant la revendication 1, dans lequel la première antenne (54) résonnante est en liaison de signal avec le dispositif (53) d'alimentation en énergie et avec le dispositif (52) de transmission de données, dans lequel la première antenne (54) résonnante a une première fréquence de résonnance et une deuxième fréquence de résonnance qui en diffère, dans lequel, pour la première antenne (54) résonnante, il y a une désadaptation sensible entre l'impédance de la liaison de signal et l'impédance de la première antenne (54) résonnante à la première fréquence de résonnance et à la deuxième fréquence de résonnance.

4. Capteur suivant la revendication 1, dans lequel le dispositif (52) de transmission de données est conçu pour moduler une énergie prélevée de la première antenne (54) résonnante.

5. Capteur suivant l'une des revendications précédentes, dans lequel le capteur a une première enveloppe (57) et la première antenne (54) résonnante et/ou la deuxième antenne (55) résonnante sont voisines de la première enveloppe (57) d'un côté du capteur, qui n'est pas tourné vers le patient (100) lors d'un fonctionnement du capteur conforme à l'utilisation.

6. Capteur suivant l'une des revendications 1 à 5, dans lequel le capteur a, distincte de la première enveloppe (57) du capteur, une deuxième enveloppe, qui est reliée par une ligne électrique à la première enveloppe (57) et dans laquelle est disposée la première antenne (54) résonnante et/ou la deuxième antenne (55) résonnante.

7. Système composé d'un tomodensitomètre (1) à résonnance magnétique et d'un capteur suivant l'une des revendications précédentes, dans lequel le tomodensitomètre (1) à résonnance magnétique a un dispositif (61) d'émission d'énergie, un dispositif (62) de réception de données et une troisième antenne (63) résonnante ayant une troisième fréquence de résonnance, dans lequel la troisième antenne (63) résonnante est en liaison de signal avec le dispositif (62) de réception de données, et la troisième fréquence de résonnance de la troisième antenne (63) résonnante coïncide sensiblement avec la première fréquence de résonnance de la première antenne (55) résonnante et, sans interaction notable entre la première antenne (54) résonnante et la troisième antenne (63) résonnante, il y a une désadaptation sensible entre l'impédance de la liaison de signal et l'impédance de la troisième antenne (63) résonnante à la troisième fréquence de résonnance,
dans lequel le système est conçu pour, par l'interaction de la première antenne résonnante avec la troisième antenne résonnante dans le champ proche, modifier l'impédance de la première antenne résonnante et inversement de la troisième antenne résonnante, de manière à ce que la désadaptation, lors de la transmission d'énergie ou de la transmission de données dans le champ proche, soit sensiblement réduite ou disparaissent complètement, et de manière à transmettre dans le champ proche de la troisième antenne résonnante, au moyen de la première antenne, des données sans pour autant émettre dans l'espace de l'énergie dans une assez grande étendue.

8. Système suivant la revendication 7, dans lequel la troisième antenne (63) résonnante est disposée dans une partie du tomodensitomètre (1) à résonnance magnétique qui, lors d'un usage conforme à l'utilisation du capteur, se trouve à proximité immédiate de la première antenne (54) résonnante.

9. Système suivant la revendication 8, dans lequel, lors d'un usage conforme à l'utilisation, il est formé des surfaces, se trouvant entre la troisième antenne (63) résonnante et la première antenne (54) résonnante d'un boîtier du tomodensitomètre (1) à résonnance magnétique et d'une enveloppe du capteur, qui sont complémentaires, de manière à rendre sensiblement minimum une distance entre la troisième antenne (63) résonnante et la première antenne (54) résonnante.

10. Système suivant l'une des revendications 7 à 9, dans lequel le système a une pluralité de premières antennes (54) résonnantes et de troisièmes antennes (63) résonnantes.
